# EUROPEAN PATENT SPECIFICATION

(11) **EP 1 680 184 B1**
(45) Date of publication and mention of the grant of the patent: **29.08.2007**
(21) Application number: 04768892.4
(22) Date of filing: 15.10.2004
(51) Int. Cl.: A61N 5/06

(54) **APPARATUS FOR ILLUMINATING A ZONE OF MAMMALIAN SKIN**
GERÄT ZUR BELEUCHTUNG EINER ZONE VON SÄUGETIERHAUT
APPAREIL SERVANT A ILLUMINER UNE ZONE DE LA PEAU D'UN MAMMIFERE

(30) Priority: 16.10.2003 GB 0324254
(43) Date of publication of application: 19.07.2006
(73) Proprietor: Chromogenex Technologies Limited, Llanelli Carmarthenshire SA14 8QG (GB)
(72) Inventor: WILLIAMS, Christopher, Edwin Euphotonics Limited, Llanelli SA14 8QG (GB)
(74) Representative: Ratcliffe, Susan Margaret
(86) International application number: PCT/GB2004/004362
(87) International publication number: WO 2005/039699

(56) References cited:
- DE-A- 10 128 629
- DE-A1- 2 740 969
- DE-U- 29 519 433
- FR-A1- 2 591 902
- GB-A- 2 356 570
- US-A- 5 616 140
- US-A- 6 063 108
- US-A- 6 074 411
- US-B1- 6 616 447

## Description

The present invention relates to an apparatus for illuminating a zone of mammalian skin.

It is well known to utilize many forms of light to improve the appearance of skin. Among lighter-skinned people, for example, ultra-violet light has been used to produce a tan, and to otherwise improve general skin color and tone. In many cultures light has been used to reduce acne and other skin blemishes. Light has also been used medically to stimulate wound healing, and to treat inflammatory conditions, skin ulcers, and wrinkles.

Acne Vulgaris is a condition of the sebaceous glands which affects 80% of individuals between the ages of 11 and 30. It is not confined to these age groups however and can affect all ages from neonates to the elderly. Factors which are considered of primary significance to the condition include an increase in the production of sebum, abnormal follicular keratinisation, the presence of Proprionibacterium and subsequent inflammation.

Dependant on the size, content, and depth of the inflamed acne lesion, it is defined as a papule (less than 0.5cms in diameter), nodule (elevated solid lesion > than 0.5cms) pustule (a papule what contains purulent material) or a cyst (nodule that contains fluid or semisolid matter).

Hair follicles are minute passages in the skin, which allow hairs to grow and produce sebum secretions from sebaceous glands which are housed within the hair follicle. Due to increased androgen levels or an excessive reaction by the sebaceous glands to androgen production, the sebaceous glands enlarge resulting in increased secretion of sebum which along with the keratinisation process of the epithelial cells, clog the hair follicle. Initially these blockages are microscopic then develop into whiteheads or blackheads (Comedones). Congested follicles are an ideal medium for growth of bacteria. When sebum levels are increased, the skin commensal proprionibacterium ingest the clogged sebum under the skin and chemicals are produced which trigger the immune system to initiate the inflammatory changes and erythematous macules associated with Acne Vulgaris.

Inflammation is the body's response to invasion of pathogens and the redness associated with the acne lesions is the result of increased blood flow whereby the white blood cells invade bacterial cells and damage tissue and produce pus. Other fluids flood to the area and collect at the site of the inflamed tissue.

The approaches which are currently used for tackling Acne Vulgaris include Drug therapies (Systemic antibiotics, cortisone injections, Dianette (women only contraceptive pill, Roaccutane, Retinoids),PUVA (Psoralen and Ultra Violet light, type A), UVB Phototherapy, Dermalux (a system using a combination of red and blue light to treat acne), Peeling agents, laser resurfacing, Dermabrasion and Microdermabrasion.

Known apparatus for treating acne by irradiation are disclosed, for example, in GB2356570,WO00/32272, WO02/32505 and US5549660. These apparatus are typically large and cumbersome and require a subject to visit a surgery for treatment.

There is a continuing need for an apparatus and method which allows administration of a low safe dose of illuminating radiation for individual need away from a doctors or cosmetic surgery.

The present invention provides an improved apparatus for illuminating a zone of mammalian skin.

The document FR-A-2 591 902 disclosed an apparatus for illuminating a zone of mammalian skin comprising a suction cup including one or more light emitters. This document thus discloses the preamble of claim 1

According to the present invention, there is provided an apparatus for illuminating a zone of mammalian skin, according to Claim 1

In the context of the invention, significant ablation of the skin may comprise a substantially deleterious effect upon a basal layer of skin. More particularly, "without producing significant ablation "preferably means that no more than 5% of the cells in the area of skin being irradiated die within 5 hours of the application of the light. It is even more preferable that no more than3%, or even 1 % of such cells die within the 5 hour window after such application and even more preferred that less than 1% of such cells die.

As used herein the term "light" is not limited to visible light. Also contemplated are other wavelengths of light including especially near UV and near IR. The term "light" is thus synonymous with the term "electromagnetic radiation".

The applicator is adapted for removable attachment to the zone of mammalian skin. The applicator comprises a suction cup and adhesion to the skin is be facilitated by provision of a replaceable hypo-allergenic adhesive ring. The inner surface of the suction cup is preferably of a light reflecting material which may be a silver or white coating or lining on the inside surface of the cup.

The adhesive may include an active material such as a topical agent that can be activated by light emitted to act on the body surface.

The apparatus preferably includes a plurality of light emitters. Preferred light emitters are light emitting diodes (LEDs) and diode lasers. The apparatus may use other light fluence sources. In the case of LEDs a single LED or a number of LEDs for example a bank of LEDs may be used. The wavelength of light emitted from the light emitter is preferably substantially in the range 400nm to 1000nm, more preferably substantially in the range 400nm to 450nm and/or 570nm to 590nm, even more preferably substantially in the range570nm to 590nm. When a plurality of light emitters are provided in accordance with a preferred embodiment of the present invention, one or more of the light emitters may emit light in a predetermined wavelength range and one or more different light emitters may emit light in a different predetermined wavelength range. For example, one or more light emitters may emit blue light (400nm to 500nm) and one or more light emitters may emit yellow light(570nm to 590nm).

The controller is preferably provided in a housing which may also include a power source. The power source is preferably one or more batteries, typically one or more rechargeable batteries. The housing is ideally a small, light, portable, pocket sized unit, typically similar in size to a personal stereo, which can be easily carried by a user, for example strapped to a belt or inserted in a pocket. The user is therefore, advantageously free to move about whilst receiving treatment from the apparatus.

The energy density of light directed toward the zone of mammalian skin is typically low intensity to avoid significant ablation of the mammalian skin. This provides that the apparatus is suitable for personal use away from a doctors or cosmetic surgery.

The controller is preferably configured to control the period of time that light is emitted from the light emitter for one treatment period. The duration of light emitted from the light emitter is preferably substantially in the range 1 minute to 1 hour, more preferably substantially in the range 20 to 40 minutes, more preferably, about 30 minutes. Desirably, the controller is configured to permit variable selection of light duration within the preferred range and/or inhibit the duration of the period of light emitted above substantially 1 hour.

The light emitted may be pulsed or continuous wave. Pulsed energy may be preferred in order to avoid overheating of the skin whilst producing the desired effect. Also by using pulsed operation, light emitting devices (particularly LED's) may be driven harder to produce more light output. A typical LED can operate at a drive current of 50mA in continuous mode, whilst in pulsed operation, for short periods, the same diode can be pulsed at current of around 200mA. Pulsed operation will also allow more diodes to be used for a given energy source requirement, thus reducing the power requirement and prolonging the life of the battery or the like used to power the light emitters.

The apparatus of the present invention is preferably used for the treatment of acne vulgaris.

The apparatus according to the present invention provides a low intensity light and is therefore suitable for home use away from a doctors or cosmetic surgery without any medical intervention.

When the apparatus of the present invention is used for the cosmetic treatment of a skin condition (particularly acne vulgaris), the light directed to the zone of mammalian skin typically is of a predetermined wavelength and is delivered according to a predetermined regime in order to have the desired effect at the zone of skin including a reaction leading to at least partial disabling or eradication of the cause of the skin condition.

A feature of the technique of the invention is that efficacy is achieved without the need of any other topically applied agent or any invasive or ablative procedure. However, it is also contemplated that the apparatus of the present invention could be used in conjunction with a topical agent (such as a retanoid) applied to the skin.

The radiation is typically low intensity (avoiding ablation at or below the skin surface) and typically primarily of wavelength at or about the wavelength of yellow light (570nm to 590nm) for reasons explained in detail later. Absorption of light is through the dermal vasculature having no adverse effects on the epidermis.

Desirably, the reaction leading to at least partial removal or disabling of the cause of the skin condition is a photochemical reaction, caused by selective absorption of the predetermined wavelength light, typically by a preselected chromophore.

For Acne Vulgaris the chromophore targeted to combat the proprionibacterium is porphyrin in the connective tissue. This tissue bound photosensitiser when excited from light of a certain wavelength (typically the wavelength of blue light 400nm to 450 nm and yellow light 570nm to 590nm), produces a photochemical reaction resulting in the production of singlet oxygen thereby destroying the bacterium.

Proprionibacterium is averse to oxygen (anaerobic) and relies upon chemicals known as porphyrins in skin tissue. Porphyrin is usually innocuous in the absence of light. It is however photosensitive and when exposed to light of the required wavelength the photochemical reaction occurs. This results in a transition from the porphyrin's ground state to a reactive triplet state. At this level, a reaction with molecular oxygen creates singlet oxygen. Through the medium of a suitable light source, to activate the porphyrins to produce singlet oxygen, the bacterium responsible for Acne Vulgaris can be cleared in a cosmetic, pain-free, non-invasive and efficient manner.

Vasodilation and hyperaemia are integral parts of the inflammatory response, including response to infection. Therefore any inflammatory/infective focus contains a disproportionate concentration of red blood cells.

Porphyrin molecules are contained in the heam of haemoglobin so that any inflammatory or infective focus contains a concentration of natural porphyrin. Activation of this porphyrin using, for example, yellow (570nm to 590nm) light releases substances which destroys adjacent toxins such as bacteria in acne.

Similarly, any acute inflammatory condition of skin such as rosaceae will be helped although the exact toxin may be unknown.

Yellow light (570nm to 590nm) is beneficially readily absorbed by the epidermis and will generally penetrate the tissue underlying the epidermis to reach the target porphyrin.

Where the skin condition is Acne Vulgaris it is therefore preferred that the wavelength of the illuminating radiation comprises a primary wavelength or narrow wavelength band substantially in the range 570-590nm.

The photochemical interaction is typically dependent upon the number of incident photons, so the photons may be delivered in pulsed or continuous wave mode.

The target for the light has to be a material that absorbs a specific wavelength and disregards other wavelengths (chromophore). In accordance with the invention, for Acne Vulgaris, the chromophores may be porphyrin in skin tissue and oxyhaemoglobin in the dermal vasculature.

The preferred wavelength (or wavelengths) for this invention will depend upon the skin condition being treated but typically include a wavelength in the range of 400nm to 1500nm with a preferred range of 400nm to 450nm and/or 570nm to 590nm, and even more preferred range of 570 to 590nm.

The invention will now be further described in an exemplary embodiments, byway of example only, with reference to the accompanying drawings in which:
Figure 1 is a schematic view of an exemplary embodiment of the apparatus of the present invention; and
Figure 2 is a schematic view of part of the apparatus of Figure 1.

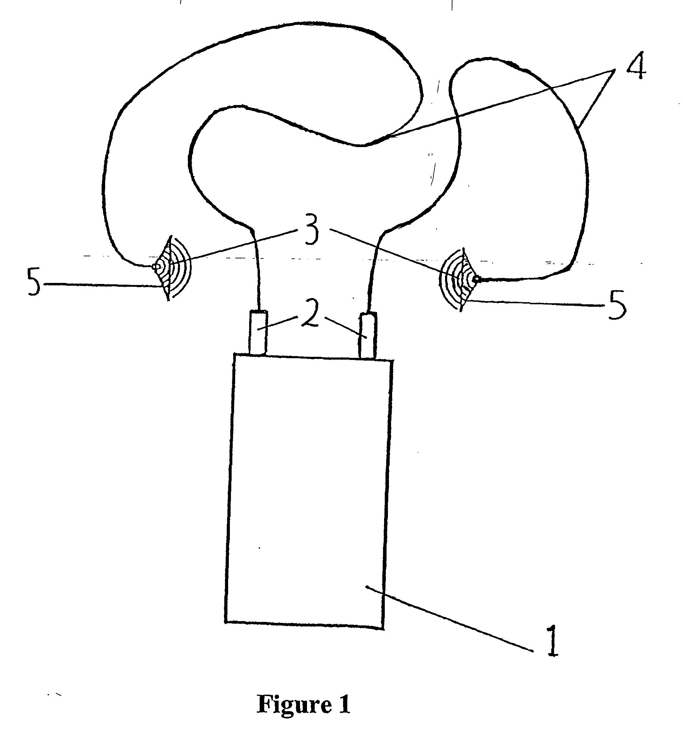

The apparatus shown in Figure 1 comprises a base unit 1 with four remote light emitters 3 (only two light emitters are shown) connected to the base unit 1 via electrical or optical leads 4.

The base unit 1 can be of any suitable size, shape, colour, material etc. It is preferred however, that the base unit 1 is a small, light, portable pocket sized unit, for example similar in size to a personal stereo. A user would therefore advantageously be able to use the apparatus at home for extended periods at their convenience, whilst moving about the home.

The base unit 1 houses a controller which regulates the duration and/or intensity of light emitted from the light emitters 3 to produce a desired effect without producing significant ablation of the mammalian skin.

A fixed resistor (not shown) may be provided to limit the current drawn by light emitters 3 and thus reduce the power required.

The controller may be a timer provided in the base unit 1 which can be set to provide output of light from the light emitters 3 for a predetermined period of time. By providing a low energy density of light for a long duration (typically 30 minutes) a desired effect, for example cosmetic treatment of a skin condition such as acne, typically results without producing significant ablation of the mammalian skin. The light emitted may be in a continuous wave or pulsed to improve absorption by allowing thermal relaxation periods in the treatment. The controller typically regulates the pulse width and duration according to a predetermined regime.

A user may desirably be able to set the controller such that the light emitted is of an energy density, duration and pulse regime according to the desired effect required and the users skin type (for example, fair or dark skinned). Alternatively, or additionally, the controller may be able to automatically set the optimal parameters typically following some input from the user, such as desired effect required and skin type. Whatever parameters are selected whether automatically or manually, the controller is suitably configured to only allow light emitted to be of a wavelength, energy density, duration and pulse regime that will not produce significant ablation of the users skin, preferably substantiallyno ablation. This provides that the apparatus is suitable for personal use away from a doctors or cosmetic surgery.
The base unit 1 may also house a power source. The power source can be of any known type, conventional or otherwise and is preferably one or more batteries which are ideally rechargeable.

The light emitters 3 may be light emitting diodes (LEDs) or diode lasers. Where lasers are used, the output may be monochromatic. Alternatively, or in the case where LED's are used, the radiation delivered may be "effectively" monochromatic (for example by means of appropriate filtering) or of a relatively narrow band width (typically within a band width of 15nm of less).

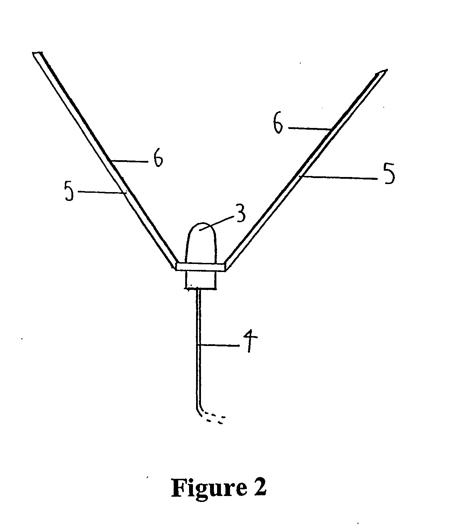

Preferably the light emitters 3 are LED's either in the blue, green, yellow or red spectrum, preferably the blue or yellow spectrum, most preferably the yellow spectrum. One or more of the light emitters 3 may emit light of first spectrum (for example, blue) whilst the other light emitters 3 may emit light of a second spectrum (for example, yellow) different from the first spectrum. The light emitters 3 are each connected to the base unit 1 by electrical or optical leads 4. The leads 4 have a plug 2 at one end and a suction cup 5 at the other end. The plug 2 can be removably plugged into base unit 1, so that a user can plug between one and four light emitters 3 into the base unit 1 depending on the treatment required. The light emitters 3 are each housed in the suction cup 5 attached to the end of the leads 4, as shown more clearly in Figure 2.

The inner surface of the suction cup 5 is lined with a reflective material 6, such as a silver or white lining. The reflective material acts to increase the light directed to the surface of the mammalian skin.

In use, one or more of the suction cups 5 can be attached to the surface of a users skin at any location of the body where treatment is required. For example, the suction cup 5 can be attached in the proximity of a spot to bathe the area in a controlled intensity of light for a predetermined period of time. Attachment to the skin is facilitated by an adhesive, such as a replaceable or consumable hypoallergenic adhesive ring.

The presently disclosed apparatus and methods have numerous applications that improve the appearance of skin, that is having a desired skin effect. Where ultra-violet light is used, for example, the improvement may involve tanning. Where light is applied at 585 nm, or other wavelength that is well absorbed by the dermis, but only poorly absorbed by the epidermis or basal layer, the improvement in skin may be in making the skin smoother. Smoother skin can be achieved, for example, through a reduction in the depth or width of a wrinkle, or through increasing collagen production under an indentation caused by acne. Another improvement may be treatment of acne, particularly acne vulgaris. Another improvement may be reduction in the-growth or presence of hair, such as where the application conditions are satisfactory to kill or at least significantly damage cells structurally and/or physiologically associated with growth of hair, and particularly cortical cells including hair follicle cells, papilla cells, outer and inner root sheath cells (e.g.: Huxley's layer and Henley's layer). Still another improvement in skin can be achieved indirectly, by affecting a medical condition, such as the treatment of skin cancers. The apparatus may be used in conjunction with a photodynamic therapy (PDT) drug, typically applied to the surface of the skin where the light is directed.

The apparatus of the inventions has a wide range of applications for affecting skin conditions such as acne which may be as a result of infection with acne vulgaris. The apparatus may be used to treat more deep seated skin conditions such as ulcers or skin lesions. However, the apparatus may be used in methods of treating the skin or body which have purely cosmetic applications such as reducing wrinkles, evening out skin tone or even the whitening of teeth.

Specific embodiments and applications of light therapy methods and apparatus have been disclosed. It should be apparent, however, to those skilled in the art that many more modifications besides those already described are possible without departing from the inventive concepts herein. The inventive subject matter, therefore, is not to be restricted except in the scope of the appended claims. Moreover, in interpreting both the specification and the claims, all terms should be interpreted in the broadest possible manner consistent with the context.

## Claims

1. An apparatus for illuminating a zone of mammalian skin, the apparatus comprising:
a) one or more light emitters;
b) an applicator movable to direct light emitted from the light emitter to the zone of mammalian skin; and
c) a controller for controlling the duration and/or intensity of light delivered to the surface of the skin to provide a desired effect without producing significant ablation of the mammalian skin;
wherein the applicator comprises a suction cup in which the one or more light emitters are housed, **characterised in that** there being a replaceable hypo- allergenic adhesive ring attached to the applicator to allow for removable attachment of the applicator to the zone of mammalian skin.

2. Apparatus according to claim 1, wherein the inner surface of the suction cup is of a light reflecting material.

3. Apparatus according to claim 1 or claim 2 wherein the applicator includes a topical agent.

4. Apparatus according to any preceding claim, wherein a plurality of light emitters are included.

5. Apparatus according to any proceeding claim, wherein the light emitter is a light emitting diode (LED) or diode laser.

6. Apparatus according to any preceding claim, wherein the wavelength of light emitted from the light emitters is substantially in the range 400nm to 1000nm.

7. Apparatus according to any preceding claim, wherein the wavelength of light emitted from the light emitters is substantially in the range 400nm to 450nm and/or 570nm to 590nm

8. Apparatus according to any preceding claim, wherein the wavelength of light emitted from the light emitters is substantially in the range 570nm to 590nm.

9. Apparatus according to any preceding claim, wherein the light emitter is received in the applicator.

10. Apparatus according to any preceding claim, wherein the controller is provided in a housing.

11. Apparatus according to any preceding claim, further including a power source.

12. Apparatus according to claim 11, wherein the power source is one or more batteries, preferably one or more rechargeable batteries.

13. Apparatus according to any preceding claim, wherein the controller is arranged to control the period of time that light is emitted from the light emitter for one treatment period.

14. Apparatus according to claim 13, wherein the duration of light emitted from the light emitter is substantially in the range 1 minute to 1 hour.

15. Apparatus according to claim 13, wherein the duration of light emitted from the light emitter is substantially in the range 20 to 40 minutes.

16. Apparatus according to claim 13, wherein the duration of light emitted from the light emitter is substantially 30 minutes.

17. Apparatus according to any of claims 14 to 16, wherein the controller is configured to permit variable selection of light duration within said range and/or inhibit the duration of the period of light emitted above a predetermined time.

## Patentansprüche

1. Vorrichtung zur Beleuchtung einer Zone von Hautgewebe eines Säugers, die Vorrichtung umfassend:
a) einen oder mehrere Lichtemitter;
b) einen Applikator, der bewegbar ist, um Licht, welches von den Lichtemittern ausgesendet wird, auf die Zone des Hautgewebes zu richten; und
c) eine Steuerung zum Steuern der Dauer und/oder Intensität des Lichts, welches auf die Oberfläche der Haut übertragen wird, um einen gewünschten Effekt ohne Erzeugung eines signifikanten Abtrags des Hautgewebes bereitzustellen, worin der Applikator eine Saugtasse umfasst, in welcher der eine oder die mehreren Lichtemitter aufgenommen ist/sind,
**dadurch gekennzeichnet, dass**
ein austauschbarer, hypoallergener klebender Ring an dem Applikator befestigt ist, um eine entfernbare Befestigung des Applikators an der Zone des Hautgewebes zu ermöglichen.

2. Vorrichtung nach Anspruch 1, worin die innere Oberfläche der Saugtasse aus einem lichtreflektierenden Material ist.

3. Vorrichtung nach Anspruch 1 oder 2, worin der Applikator einen topischen Wirkstoff beinhaltet.

4. Vorrichtung nach einem der vorhergehenden Ansprüche, worin eine Vielzahl von Lichtemittern beinhaltet sind.

5. Vorrichtung nach einem der vorhergehenden Ansprüche, worin der Lichtemitter eine lichtemittierende Diode (LED) oder ein Diodenlaser ist.

6. Vorrichtung nach einem der vorhergehenden Ansprüche, worin die Wellenlänge des von den Lichtemittern emittierten Lichtes im wesentlichen im Bereich von 400nm bis 1000nm ist.

7. Vorrichtung nach einem der vorhergehenden Ansprüche, worin die Wellenlänge des von den Lichtemittern emittierten Lichtes im wesentlichen im Bereich von 400nm bis 450nm und/oder 570nm bis 590nm ist.

8. Vorrichtung nach einem der vorhergehenden Ansprüche, worin die Wellenlänge des von den Lichtemittern emittierten Lichtes im wesentlichen im Bereich von 570nm bis 590nm ist.

9. Vorrichtung nach einem der vorhergehenden Ansprüche, worin der Lichtemitter in dem Applikator aufgenommen ist.

10. Vorrichtung nach einem der vorhergehenden Ansprüche, worin die Steuerung in einem Gehäuse bereitgestellt ist.

11. Vorrichtung nach einem der vorhergehenden Ansprüche, weiterhin beinhaltend eine Energiequelle.

12. Vorrichtung nach Anspruch 11, worin die Energiequelle eine oder mehrere Batterien ist, vorzugsweise eine oder mehrere wiederaufladbare Batterien.

13. Vorrichtung nach einem der vorhergehenden Ansprüche, worin die Steuerung ausgebildet ist, um die Zeitperiode zu steuern, in welcher Licht von dem Lichtemitter für eine Behandlungsperiode emittiert wird.

14. Vorrichtung nach Anspruch 13, worin die Dauer des von dem Lichtemitter emittierten Lichtes im Wesentlichen im Bereich von einer Minute bis einer Stunde ist.

15. Vorrichtung nach Anspruch 13, worin die Dauer des von dem Lichtemitter emittierten Lichtes im Wesentlichen im Bereich von 20 bis 40 Minuten ist.

16. Vorrichtung nach Anspruch 13, worin die Dauer des von dem Lichtemitter emittierten Lichtes im Wesentlichen 30 Minuten ist.

17. Vorrichtung nach einem der Ansprüche 14 bis 16, worin die Steuerung ausgebildet ist, um eine variable Auswahl der Lichtdauer innerhalb des Bereichs erlaubt und/oder die Periodendauer des emittierten Lichts oberhalb einer vorbestimmten Zeit verhindert.

## Revendications

1. Appareil servant à illuminer une zone de peau d'un mammifère, l'appareil comprenant :
a) un ou plusieurs émetteurs de lumière ;
b) un applicateur mobile pour diriger la lumière émise à partir de l'émetteur de lumière sur la zone de peau d'un mammifère ; et
c) un dispositif de commande pour commander la durée et/ou l'intensité de la lumière délivrée sur la surface de la peau pour fournir un effet souhaité sans produire une ablation importante de la peau d'un mammifère ; dans lequel l'applicateur comprend une ventouse dans lequel un ou plusieurs émetteurs de lumière sont logés, **caractérisé en ce qu'**il y a un anneau adhésif hypoallergénique remplaçable fixé à l'applicateur pour permettre à la fixation amovible de l'applicateur d'aller sur la zone de peau d'un mammifère.

2. Appareil selon la revendication 1, dans lequel la surface interne de la ventouse est constituée d'un matériau de réflexion de la lumière.

3. Appareil selon la revendication 1 ou la revendication 2, dans lequel l'applicateur comprend un agent topique.

4. Appareil selon l'une quelconque revendication précédente, dans lequel une pluralité d'émetteurs de lumière est comprise.

5. Appareil selon l'une quelconque revendication précédente, dans lequel l'émetteur de lumière est une diode électroluminescente (LED) ou un laser à diode.

6. Appareil selon l'une quelconque revendication précédente, dans lequel la longueur d'onde de la lumière émise depuis les émetteurs de lumière est sensiblement comprise entre 400 nm et 1000 nm.

7. Appareil selon l'une quelconque revendication précédente, dans lequel la longueur d'onde de la lumière émise à partir des émetteurs de lumière est sensiblement comprise entre 400 nm et 450 nm et/ou entre 570 nm et 590 nm.

8. Appareil selon l'une quelconque revendication précédente, dans lequel la longueur d'onde de la lumière émise depuis les émetteurs de lumière est sensiblement comprise entre 570 nm et 590 nm.

9. Appareil selon l'une quelconque revendication précédente, dans lequel l'émetteur de lumière est reçu dans l'applicateur.

10. Appareil selon l'une quelconque revendication précédente, dans lequel le dispositif de commande est fourni dans un logement.

11. Appareil selon l'une quelconque revendication précédente, comprenant en outre une source d'énergie.

12. Appareil selon la revendication 11, dans lequel la source d'énergie est une ou plusieurs piles, de préférence une ou plusieurs piles rechargeables.

13. Appareil selon l'une quelconque revendication précédente, dans lequel le dispositif de commande est disposé pour commander la période de temps durant laquelle la lumière est émise à partir de l'émetteur de lumière pour une période de traitement.

14. Appareil selon la revendication 13, dans lequel la durée de la lumière émise à partir de l'émetteur de lumière est sensiblement comprise entre 1 minute et 1 heure.

15. Appareil selon la revendication 13, dans lequel la durée de la lumière émise à partir de l'émetteur de lumière est sensiblement comprise entre 20 et 40 minutes.

16. Appareil selon la revendication 13, dans lequel la durée de lumière émise à partir de l'émetteur de lumière est sensiblement de 30 minutes.

17. Appareil selon l'une quelconque des revendications 14 à 16, dans lequel le dispositif de commande est configuré pour permettre une sélection variable de durée de la lumière dans ladite plage et/ou inhibe la durée de la période de lumière émise sur un temps prédéterminé.
